## Europäisches Patentamt

## European Patent Office

## Office européen des brevets

(11) Veröffentlichungsnummer: **0 252 101 B1**

# EUROPÄISCHE PATENTSCHRIFT

(45) Veröffentlichungstag der Patentschrift: **03.06.92**

(21) Anmeldenummer: **87900071.9**

(22) Anmeldetag: **20.12.86**

(86) Internationale Anmeldenummer:
**PCT/DE86/00529**

(87) Internationale Veröffentlichungsnummer:
**WO 87/03814 (02.07.87 87/14)**

(51) Int. Cl.⁵: **A61M 5/14**, A61F 2/02

(54) **VORRICHTUNG ZUR KONTINUIERLICHEN ODER DISKONTINUIERLICHEN VERABREICHUNG VON INSULIN IN DEN MENSCHLICHEN KÖRPER.**

(30) Priorität: **20.12.85 DE 3545260**

(43) Veröffentlichungstag der Anmeldung:
**13.01.88 Patentblatt 88/02**

(45) Bekanntmachung des Hinweises auf die
Patenterteilung:
**03.06.92 Patentblatt 92/23**

(84) Benannte Vertragsstaaten:
**AT CH DE FR GB IT LI NL SE**

(56) Entgegenhaltungen:
**EP-A- 0 098 592      EP-A-00 405 92**
**FR-A- 2 298 832      FR-A- 2 350 107**
**FR-A- 2 387 659      FR-A- 2 409 107**
**FR-A- 2 557 445**

(73) Patentinhaber: **SCHREZENMEIR, Jürgen**
**Alfred-Mumbächer-strasse 57**
**W-6500 Mainz 1(DE)**

(72) Erfinder: **SCHREZENMEIR, Jürgen**
**Alfred-Mumbächer-strasse 57**
**W-6500 Mainz 1(DE)**

(74) Vertreter: **Pöhner, Wilfried Anton, Dr.**
**Kaiserstrasse 27 Postfach 63 23**
**W-8700 Würzburg 1(DE)**

**Beschreibung**

Die Erfindung betrifft eine Vorrichtung gemäß dem Oberbegriff des Anspruches 1.

Weit verbreitet und deshalb allgemein bekannt ist die Zuckerkrankheit (Diabetes mellitus), bei welcher das Hormon der Bauchspeicheldrüse, das Insulin, überhaupt nicht oder nicht in ausreichender Menge produziert wird.

Steht das Insulin nicht in ausreichender Menge zur Verfügung wird der mit der Nahrung zugeführte Zucker nicht verbrannt, so daß die Zuckerkonzentration im Blut ansteigt und der überschüssige Blutzucker mit dem Harn ausgeschieden wird. Zur Therapie werden dem Diabetiker die fehlenden Mengen an Insulin entweder kontinuierlich durch Infusion oder aber auch diskontinuierlich durch Injektionen zugeführt, wobei man die exakt fehlende Menge zu verabreichen bemüht ist, was anhand des Blutzuckerspiegels nachgeprüft und überwacht werden kann. Eine zu geringe Insulindosis ist ebenso unerwünscht wie eine zu hohe verabreichte Menge, die zu gefährlichen Erscheinungen ("Insulin-Schock") führen kann.

Aus der FR-A-2 387 659 ist eine Einrichtung bekannt, mit deren Hilfe eine kontinuierliche Messung des Blutzuckerwertes als auch anhand Vergleiches mit dem Ist-Wert die optimal zu injizierende Menge ermittelt und dem Patient sofort verabreicht wird. Man erhält demnach eine kontinuierliche und sofortige Angleichung an den Ist-Wert. Der entscheidende Nachteil kontinuierlicher Überwachung ist die Notwendigkeit der stationären Unterbringung, was dem Patienten zur Führung eines normalen Lebens außerstande setzt.

Zur Vermeidung der damit verbundenen erheblichen Unannehmlichkeiten überwiegt die ambulante Behandlung, bei welcher dem Patienten das Insulin diskontinuierlich verabreicht wird, dessen Menge anhand der späteren Reaktionen, insbesondere den Abweichungen des Blutzuckerwertes von dem Ist-Wert, durch Abschätzung und damit sehrunpräzise in ihrer Dosierung geändert wird. Bei einer anderen noch praktizierten Möglichkeit wird die optimale Insulindosis einmal festgestellt und die dementsprechende Menge täglich verabreicht. Beide Methoden der Dosierung der Insulinmenge führen bereits deshalb schon zu ungenauen Ergebnissen, weil im ersten Fall die ermittelte Menge recht unpräzise ist und im zweitgenannten die sich über einen gewissen Zeitraum in aller Regel verändernden optimalen Dosierung von der ursprünglich eingestellten abweicht. In nachteiliger Weise kommt hinzu, daß die benötigte Insulinmenge von Patient zu Patient unterschiedlich und deshalb kaum vorhersagbaren Schwankungen unterworfen ist.

Hiervon ausgehend hat sich die Erfindung die Schaffung einer Vorrichtung zur Aufgabe gemacht, mit deren Hilfe bei diskontinuierlicher Verabreichung und unter Berücksichtigung von über Mahlzeiten zugeführte Kohlenhydratmengen eine präzise Bestimmung der Insulingabe möglich wird.

Gelöst wird diese Aufgabe erfindungsgemäß durch eine Vorrichtung, die sich durch die im Kennzeichen des Hauptanspruches angegebenen Merkmale auszeichnet.

In Abkehr von der bisherigen Praxis, neben dem durch Unterfunktion der Bauchspeicheldrüse benötigten Grundbedarf, der durch lange wirksame Insulinmengen, und der mahlzeitbedingte Bedarf, der durch kurzwirkende Insulingaben abgedeckt zu werden pflegt, ermittelt die erfindungsgemäße Vorrichtung nahezu exakt diejenige Insulingabe, die unter Berücksichtigung sämtlicher Faktoren u.a. auch für die Verbrennung der Speisen notwendig ist. Die am Tage I aufgrund der durch die Mahlzeit M aufzunehmende Insulingabe $IBO (M,I)$ bestimmt sich durch das Produkt der in der Mahlzeit enthaltenen Kohlenhydrate $= COH (M,I)$ sowie dem zugehörigen Insulin-Kohlenhydratäquivalent $ICOHÄ (M,I)$. Der Wert der Kohlenhydratmenge $COH(M,I)$ schwankt und ist in Abhängigkeit von der jeweils eingenommenen Mahlzeit einzusetzen, wobei keine grundsätzlichen Schwierigkeiten bestehen anhand der Zusammensetzung der Mahlzeit die aufgenommene Menge an Kohlenhydraten = Broteinheiten zu ermitteln. Schwierigkeiten ergeben sich jedoch insbesondere daraus, daß das Insulin-COH-Äquivalent $ICOHÄ (M,I)$ nicht nur von Patient zu Patient großen Schwankungen unterworfen ist, sondern zudem über den Tag verteilt (Frühstück, Mittag- und Abendessen) in zyklischer Weise unterschiedliche Werte annimmt.

Hierbei gibt der Index M die jeweilige Mahlzeit an, also z.B. Frühstück, Mittag- oder Abendessen. In aller Regel wird bei Diabetikern die Nahrungsaufnahme nicht auf 3 sondern üblicherweise auf 5-6 Mahlzeiten pro Tag verteilt. $(M + 1)$ bezeichnet die darauffolgende Mahlzeit. Der Index I bezeichnet den momentan betrachteten Tag, also ist $(I-1)$ der Vortag. Zunächst wird die am Vortag $(I-1)$ zur gleichen Mahlzeit M verabreichte Insulingabe $IBO (M,I-1)$ gespeichert. Von diesem Wert wird die Korrektur-Insulinmenge $KORI (M,I-1)$ subtrahiert, welche dem Anteil entspricht, der in der Insulingabe enthalten ist zum Ausgleich eines aufgrund einer am Vortage $(I-1)$ vor der Mahlzeit M durchgeführten Blutzuckermessung festgestellten Defizits. Der Wert $KORI (M,I-1)$ stellt also den Korrekturwert dafür dar, daß noch vor Einnahme der Mahlzeit bereits ein Blutzuckerdefizit vorhanden war.

Zu diesem, durch Subtraktion erhaltenen Ergebnis wird diejenige Korrekturinsulinmenge addiert, die sich daraus ergibt, daß am Vortag $(I-1)$ zu Beginn der darauffolgenden $(M + 1)$-ten Mahlzeit aufgrund der Messung des Blutzuckers ein Defizit festgestellt wurde. Diese Menge stellt ein unmittelbares Maß für die Abweichung bzw. Exaktheit und Genauigkeit der zur Verbrennung der mit der M-ten Mahlzeit eingenommenen Kohlenhydratmengen erforderlichen Insulinmenge dar, die tatsächlich erforderlich gewesen wäre. Das auf diesem Wege erhaltene Ergebnis wird durch die am Vortage (I-1) mit der Mahlzeit M eingenommene Broteinheitenmenge dividiert und ergibt das am heutigen, I-ten Tage zur Mahlzeit M der der Berechnung zugrunde zu legenden Insulin-Kohlenhydrat-Äquivalent an.

Bei der erfindungsgemäß vorgeschlagenen Berechnungsmethode finden einzig und allein die Werte des Vortages Berücksichtigung. In einer Weiterbildung ist vorgesehen, daß das gesuchte Insulin-COH-äquivalent ICOHÄ (M,I) am I-ten Tag zur M-ten Mahlzeit als Mittelwert berechnet wird aus mehreren davor befindlichen Tagen. Die Art und Weise der Berechnung des Mittelwertes (arithmetisch, geometrisch usw.) steht hierbei grundsätzlich frei, insbesondere können die ermittelten Werte der unmittelbar vor dem interessierenden Tag errechneten Insulin-COH-Äquivalente mit höherem Gewicht berücksichtigt werden um die von Tag zu Tag verbesserte Anpassung zu berücksichtigen und andererseits die Konvergenz zum Idealwert hin zu beschleunigen.

Die angegebene mathematische Beziehung zur Berechnung der Insulingabe bezieht sich auf die am Vortage (I-1) verabreichten Insulingaben und die entsprechenden Blutzuckermessungen. Diese Formel ist deshalb zur Bestimmung der Insulingabe bei der M-ten Mahlzeit am 1. Tag ungeeignet, so daß man entweder wie bisher auf Schätzungen angewiesen ist oder von der in Anspruch 5 angegebenen Formel ausgeht. Diese Beziehung gibt unmittelbar die Insulingabe und nicht wie bei den vorhergehenden Berechnungen das Insulin-COH-Äquivalent wieder. Als Voraussetzung benötigt die Ermittlung nach dieser Formel den vorherigen Insulin Tagesbedarf DIN, der dividiert wird durch die am gleichen Tag verzehrten Kohlenydratmenge. Dieser den gesamten Tagesverlauf summarisch wiedergegebene Wert wird durch den Faktor 0,45 zunächst reduziert sowie um den Faktor b(M) erhöht, welcher den unterschiedlichen Insulinbedarf bei jeder Mahlzeit entsprechend dem Tagesverlauf berücksichtigt. Faktor b(M) beträgt beim Frühstück 1,5 beim Mittagessen 1,0 und beim Abendessen 1,2.

Dieser näherungsweise erhaltene Wert stellt von der Dimension her ein Insulin-Kohlenhydratäquivalent dar, welches noch mit der am 1. Tag zur M-ten Mahlzeit verzehrten Menge an Kohlenhydraten (in Gramm oder Broteinheiten) zu multiplizieren ist. Man erhält dann den Anteil der Insulingabe, durch die die aufgenommene Nahrung gedeckt bzw. auf sie zurückzuführen ist.

Es addiert sich die Korrekturinsulinmenge, die zu Beginn der M-ten Mahlzeit am 1. Tag aufgrund der Abweichungen es Blutzuckers vom Soll-Wert zusätzlich noch zu verabreichen ist. Damit setzt sich die zu verabreichende Insulingabe aus einem mahlzeitbezogenen, schätzungsweise ermittelten Anteil sowie einem Korrekturglied zum Abgleich des Ist-vom Soll-Wert zusammen.

Bislang bezogen sich die konkreten Berechnungen einzig auf die Ermittlung des Insulinbedarfes zur Verbrennung der durch die Mahlzeit aufgenommenen Kohlenhydrate (Gramm oder Broteinheiten). Ein zusätzlicher Anteil des Insulinbedarfes rührt her aus dem durch Unterfunktion der Bauchspeicheldrüse herrührenden Fehlbedarf. Der Nüchternbedarf wird in der Regel durch langwirkendes, der durch die Einnahmen der Speisen hervorgerufene Bedarf durch kurzwirkendes Insulin abgedeckt. Zur Bestimmung des Basalbedarfes schlägt nun die Erfindung vor, daß allein die im nüchternen Zustand erhaltenen Blutzuckerwerte Berücksichtigung finden. Dies ist beispielsweise morgens der Fall oder bei Verabreichung von kurzwirkenden Insulingaben nach hinreichend langem Zeitabstand, so daß keinerlei Verfälschungen des Meßergebnisses mehr zu erwarten sind.

Zwar ist der Basalbedarf in erster Näherung über den gesamten Tag verlaufend in etwa konstant, so daß ein einziger Meßpunkt, z.B. morgens zur Ermittlung des gewünschten Wertes ausreichen würde. Genauere Untersuchungen haben gezeigt, daß die Konstanz des Basalbedarfes über 24 Stunden verteilt nicht exakt ist sondern relativ konstante Schwankungen aufweist. So ist der Bedarf während der Nachtzeit geringer als tagsüber. In den frühen Morgenstunden jedoch am höchsten. Messungen haben gezeigt, daß der Verlauf des Nüchternbedarf sich innerhalb 24 Stunden ändert, also Zyklen durchläuft. Die Erfindung schlägt deshalb vor, mehrere (im nüchternen Zustand) druchgeführte Blutzuckermessungen über den Tag vorzunehmen, hieraus ein Basalbedarfsprofil ( = Verhältnis der Insulininfusionsraten zueinander) aufzustellen, anhand dessen dann in den zukünftigen Tagen im voraus der Insulinbedarf berechnet wird. Die Ermittlung des Profiles ist bei jedem Patienten erneut erforderlich, da es bei unterschiedlichen Patienten stark voneinander abweichen kann. Selbstverständlich erfordern die mit Beginn der Messung erhaltenen Werte eine Korrektur im Hinblick auf die bereits verabreichten und im Körper befindlichen Insulinmengen.

Die weitere Anpassung des Nüchterninsulinbedarfs erfolgt - unter Wahrung der Relation der Insulininfusionsraten zueinander - durch Berücksichtigung aktuell gemessener Nüchternblutzuckerwerte.

Zum ermittelten Basalbedarf addiert sich dann der zur Verbrennung der Kohlenhydrate beispielsweise nach oben genannten Formel berechneter Anteil.

Die Ermittlung des Blutzuckerwertes erfordert stets die Entnahme sowie die Analyse einer Blutmenge.

Diese Meßmethode ist aufwendig und für die ambulante Behandlung von Diabetikern nicht zufriedenstellend. Ein weiterer bedeutender Gedanke der Erfindung besteht deshalb in der Ermittlung der Blutzuckerwerte durch Messung des Urinzuckers. Dies ist deshalb möglich, weil mit Hilfe eines Umrechnungsfaktors, dem sogenannten Urin-/Blutzuckeräquivalent, ziemlich exakt aus dem Meßwert des Urinzuckers der zugehörige Blutzucker rechnerisch bestimmt werden kann. Hierzu bedarf es über eine gewisse Zeit der gleichzeitigen Messung sowohl des Urin- als auch Blutzuckers, woraus sich patientenabhängig und individuell das zur Berechnung erforderliche Urin-/Blutzuckeräquivalent bestimmen läßt. Die Messung des Urinzuckers ist wesentlich einfacher, unproblematischer und kann von jedem Patienten selbst ohne weiteres vorgenommen werden. Allerdings ist diese Art der indirekten Messung des Blutzuckers für geringfügig erhöhte Blutzuckerwerte nicht geeignet, da dann die Nierenschwelle noch nicht erreicht und über den Urin kein Zucker abgeschieden wird. In aller Regel wird durch den Verlauf des Blutzuckerwertes nach der Nahrungsaufnahme zumindest teilweise diese Schwelle überschritten, so daß sich zumindest während dieser Phasen der Erfindungsgedanke realisieren läßt.

Weitere Möglichkeiten des Einsatzes ergeben sich bei Verwendung eines Rechners (Computer) in Zusammenhang mit der Steuerung der Dosiereinrichtung oder der Anzeige. Dieser ist in vielfacher Hinsicht benutz- und einsetzbar. So kann das Abklingen der Wirkung von bereits verabreichtem Insulin in den Rechner eingegeben werden und so in den in beliebiger Zeit darauf vorgenommenen Meßungen exakt Berücksichtigung finden.

Eine weitere, zusätzlich oder unabhängig davon realisierbare Möglichkeit besteht darin, den sich entsprechend dem Tag-Nachtzyklus ändernden Insulinbedarf durch eine entsprechende Programmierung einzugeben, so daß bei der Ermittlung der benötigten Insulindosis dieser Einfluß Eingang findet.

Schließlich wird hierdurch noch die Möglichkeit eröffnet, bereits bekannte oder noch zu entwickelnde Formeln in den Rechner einzugeben, mit deren Hilfe an Hand der gemessenen Blutzuckerwerte eine weitere Verbesserung der zu ermittelnden Insulindosis möglich ist.

Neben der Aufnahme von Kohlehydraten beispielsweise durch die Nahrung ist bekannt, daß sich die benötigte Insulindosis auch durch körperliche Aktivitäten ändert. Die hierdurch bedingten Einflüße sind abschätzbar und, wie in einer Weiterbildung der Erfindung vorgesehen, mit Hilfe einer in dem Sinne auf die Steuerung der Dosierung einwirkenden Eingabe berücksichtigbar, daß die abgegebene Insulindosis entsprechend erhöht (und auch erniedrigt) wird. Es lassen sich dann auf diesem Wege die durch äußere Einflüsse auftretenden, vom üblichen Zyklus abweichenden Änderungen des Insulinbedarfs in die Bemessung der optimalen Insulindosis einbringen.

Die Erfindung stellt eine Vorrichtung zur Verfügung, mit deren Hilfe eine präzise Bemessung der vom Patienten benötigten Insulindosis möglich wird.

## Patentansprüche

1. Vorrichtung zur Bestimmung der Menge an Insulin zur diskontinuierlichen Verabreichung für die Verbrennung aufgenommener Kohlenhydrate, mit einer Meßeinrichtung zur Bestimmung des Blutzuckerwertes, einer Dosiereinrichtung zur Bemessung oder einer Anzeige zur Angabe der optimalen Insulingabe einem Regelkreis, der die Blutzuckerwerte mit den Sollwerten vergleicht, und entsprechend der hierbei erhaltenen Differenz ein Steuersignal, das die Insulingabe darstellt, an die Dosiereinrichtung oder die Anzeige abgibt, wobei der Regelkreis derart gestalltet ist, daß die im zeitlichen Abstand gemessenen Blutzuckerwerte ebenso wie die verabreichten Insulingaben gespeichert werden, und mit einer Eingabevorrichtung, durch die die bei der Mahlzeit M aufgenommene hohe Kohlenhydratmence COH (M,I) am Tage (I) eingebbar ist, **dadurch gekennzeichnet,** daß der Regelkreis derart gestalltet ist, daß sich die Insulingabe am Tage (I) zur M-ten Mahlzeit IBO (M,I) ermittelt aus dem Produkt COH (M,I) und dem Insulin-Kohlenhydratäquivalent ICOHÄ (M,I), welches sich für die M-te Mahlzeit am I-ten Tag ergibt aus der Formel:

$$ICOHÄ(M,I) = \frac{IBO(M,I-1) - KORI(M,I-1) + KORI(M+1,I-1)}{COH(M,I-1)}$$

wobei IBO (M,I-1) die Insulingabe am Vortag zur M-ten Mahlzeit ist,
KORI (M, I-1) die Korrekturinsulinmenge aufgrund des durch Messung des Blutzuckervertes unmittelbar vor der M-ten Mahlzeit am Vortage ermittelten Abweichung des Blutzuckers vom Sollwert unter Verwendung eines sog. Insulin-Blutzucker-Äquivalents,

COH (M,I-1) die Kohlenhydratmenge der zur M-ten Mahlzeit am Vortag verzehrten Speisen, KORI (M + 1,I-1) die Korrektur-Insulinmenge, die am Vortag zu Beginn der (M + 1)-Mahlzeit durch Abweichungen des Blutzuckerwertes vom Sollwert verabreicht wurde.

2. Vorrichtung nach Anspruch 1, **dadurch gekennzeichnet,** daß sich der Wert des Insulin-Kohlenhydratäquivalents ICOHÄ (M, I) am I-ten Tag zur M-ten Mahlzeit als Mittelwert der M-ten Mahlzeiten mehrerer davor befindlicher Tage evtl. mit unterschiedlichem Gewicht berücksichtigt, berechnet wird.

3. Vorrichtung nach Anspruch 1 oder 2, **dadurch gekennzeichnet,** daß sich die Insulingabe IBO (M,1) am ersten Tag zur M-ten Mahlzeit berechnet nach der Formel:

$$IBO(M,1) = \frac{0,45 \; DIN}{COH} \text{-------} b(M) \; COH(M,1) + KORI(M,1)$$

wobei DIN der tägliche Insulinbedarf an den Vortagen,COH die an den gleichen Tagen insgesamt verzehrte Kohlenhydratmenge,

b(M) = 1,5 beim Frühstück, 1,0 zum Mittag- und 1,2 zum Abendessen,

COH (M,1) ist die am ersten Tag zur M-ten Mahlzeit verzehrte Kohlenhydratmenge,

KORI(M,1) die Korrekturmenge an Insulin durch Messung des Blutzuckers unmittelbar vor der M-ten Mahlzeit.

4. Vorrichtung nach einem der Ansprüche 1 bis 3, **dadurch gekennzeichnet,** daß zur Bestimmung des aktuellen Basalbedarfs die Blutzuckerwerte im nüchternen Zustand unter Verwendung des Insulin-Blutzucker-Äquivalents quantitativ berechnet wird.

5. Vorrichtung nach Anspruch 4, **dadurch gekennzeichnet,** daß durch mehrere über den Tag verteilte Blutzuckermessungen ein Basalbedarfsprofil erstellt wird, aufgrund dessen der zukünftige Basalbedarf ermittelt wird.

6. Vorrichtung nach einem der Ansprüche 1 bis 5, **dadurch gekennzeichnet,** daß die Blutzuckerwerte durch Messung des Urinzuckers mit Hilfe eines Urin-/Blutzucker-Äquivalents berechnet werden, welches durch Messung gleichzeitig von Urin- und Blutzucker patientenbezogen ermittelt ist.

7. Vorrichtung nach Anspruch 1 bis 6, **gekennzeichnet durch** einen Rechner zur Steuerung der Dosiereinrichtung oder der Anzeige.

8. Vorrichtung nach einem der Ansprüche 1 bis 7, **gekennzeichnet durch** eine auf die Steuerung der Dosiereinrichtung oder der Anzeige wirkende Eingabe.

## Claims

1. Device for the determination of the quantity of insulin for discontinuous administration required to burn ingested carbohydrates, having a measuring system for the determination of blood-sugar values, a measuring system for calculation or an indicator for the indication of the optimum insulin dosage, a control circuit which compares the blood-sugar values with set values and which in correspondence with the difference obtained delivers a control signal representing the insulin dosage to the measuring device or indicator, whereby the control circuit is designed in such a way that the blood-sugar values as well as the administered insulin dosage, measured at intervals in time, are stored, and having an input device, by means of which the high carbohydrate quantity CON (M, I) ingested at meal M on day (I) can be entered, **wherein** said control circuit is designed in such a way that the insulin dosage on day (I) at meal M IBO (M, I) is determined from the product COH (M, I) and the insulin carbohydrate equivalent ICOHE (M, I), which is yielded for meal M on day I by the formula:

$$ICOHE(M, I) = \frac{IBO(M, I-1)-KORI(M, I-1)+KORI(M+1, I-1)}{COH(M, I-1)}$$

whereby IBO (M, I-1) is the insulin dosage on the day before at meal M,
KORI (M, I-1) the correction insulin quantity resulting from the deviation of the blood-sugar from the set value established by measuring the blood-sugar value immediately before meal M on the day before with use of a so-called insulin blood sugar equivalent,
COH (M, I-1) the carbohydrate quantity of the food consumed at meal M on the day before,
KORI (M + 1 I-1) the correction insulin quantity which was administered the day before at the beginning of meal (M + 1) due to the deviation of the blood-sugar value from the set value.

2. Device according to claim 1 **wherein** the value of of said insulin carbohydrate equivalent ICOHE (M, I) on day 1 at meal M is calculated as the mean value of meals M from several previous days, possibly taking into account different weight.

3. Device according to claim 1 or 2 **wherein** insulin dosage IBO (M, 1) on the first day at meal M is calculated according to the formula:

$$IBO(M,1) = \frac{0.45 \ DIN}{COH}b(M) \ COH(M, 1)+KORI(M, 1)$$

whereby DIN is the daily insulin demand on the days before, COH the total quantity of carbohydrates consumed on the same days,
b(M) = 1.5 at breakfast, 1.0 at lunch and 1.2 at dinner, COH (M, 1) is the quantity of carbohydrates consumed on the first day at meal M,
KORI (M, 1) the correction quantity of insulin obtained through measurement of blood-sugar immediately before meal M.

4. Device according to one of the claims 1 to 3 **wherein** to determine the current basal demand blood-sugar values are calculated quantitatively in a fasting condition using the insulin blood-sugar equivalent.

5. Device according to claim 4 **wherein** a basal demand profile is drawn up by means of several blood-sugar measurements spread over the day and through which future basal demand is determined.

6. Device according to one of the claims 1 to 5 **wherein** blood-sugar values are calculated by measuring urine sugar with the aid of an urine sugar/blood-sugar equivalent, which is determined from patient to patient through simultaneous measurement of urine sugar/blood sugar.

7. Device according to claim 1 to 6 **wherein** a computer is used to control said measuring system or said indicator.

8. Device according to one of claims 1 to 7 **wherein** an input influences the control of said measuring system or said indicator.

**Revendications**

1. Dispositif de détermination de la quantité d'insuline en administration discontinue qui sert à brûler les glucides absorbés, avec un dispositif de mesure pour la détermination du taux de glucose dans le sang, un dispositif de dosage pour la mesure ou un indicateur qui montre la dose d'insuline optimale, un circuit de régulation qui compare les taux de glucose dans le sang avec les valeurs prescrites et qui transmet, selon la différence ainsi obtenue, un signal de commande, représentant la dose d'insuline, au dispositif de dosage ou à l'indicateur, le circuit de régulation étant agencé d'une façon telle que les taux de glucose dans le sang mesurés par intervalles de temps ainsi que les doses d'insuline administrées sont mémorisés, et avec un dispositif d'entrée qui permet d'entrer la quantité élevée de glucides COH (M,I) absorbée le jour (I) au repas M, caractérisé en ce que le circuit de régulation est agencé de telle sorte que la dose d'insuline au jour (I) au M'ième repas IBO (M,I) est calculée par le produit COH (M,I) et l'équivalent insuline-glucides ICOHE (M,I), qui pour le M-ième repas le I-ième jour résulte de la formule:

$$ICOHE(M,I) = \frac{IBO(M,I-1) - CORI(M,I-1) + CORI(M+1,I-1)}{COH(M,I-1)}$$

IBO (M,I-1) étant la dose d'insuline au jour prédédent au M-ième repas,
CORI (M,I-1) étant la quantité correctrice d'insuline basée sur la différence, déterminée le jour précédent immédiatement avant le M-ième repas par mesure du taux de glucose dans le sang, entre le taux de glucose dans le sang et la valeur prescrite, en utilisant un équivalent dit insuline-glycémie,
COH (M,I-1) étant la quantité de glucides des aliments absorbés au M-ième repas le jour précédent,
CORI (M + 1,I-1) étant la quantité correctrice d'insuline qui a été administrée le jour précédent au début du (M + 1)-ième repas du fait de la différence du taux de glucose dans le sang par rapport au taux prescrit.

2. Dispositif selon la revendication 1 caractérisé en ce que la valeur de l'équivalent insuline-glucides ICOHE (M,I) le I-ième jour au M-ième repas est calculée comme valeur moyenne des M-ièmes repas de plusieurs jours précédents, le cas échéant affectés de pondérations différentes.

3. Dispositif selon la revendication 1 ou 2 caractérisé en ce que la dose d'insuline IBO (M,1) le premier jour au M-ième repas est calculée d'après la formule:

$$IBO(M,1) = \frac{0,45\ DIN}{COH}b(M)\ COH(M,1) + CORI(M,1)$$

dans laquelle:
DIN représente la demande quotidienne d'insuline aux jours précédents, COH la quantité totale de glucides absorbée aux mêmes jours,
b(M) = 1,5 au petit déjeuner, 1,0 au déjeuner et 1,2 au dîner,
COH (M,1) est la quantité de glucides absorbée le premier jour au M-ième repas,
CORI(M,1) la quantité correctrice d'insuline par mesure du taux de glucose dans le sang directement avant le M-ième repas.

4. Dispositif selon l'une des revendications 1 à 3 caractérisé en ce que les taux de glucose dans le sang à jeun sont calculés de façon quantitative à l'aide de l'équivalent insuline-glucose pour la détermination de la demande basale actuelle.

5. Dispositif selon la revendication 4 caractérisé en ce qu'un profil de la demande basale, au moyen duquel la demande basale ultérieure est déterminée, est établi à partir de plusieurs mesures du glucose dans le sang réparties sur la journée.

6. Dispositif selon l'une des revendications 1 à 5 caractérisé en ce que les taux de glycémie sont calculés à partir de mesures du taux de glucose dans les urines et à l'aide d'un équivalent taux de glucose urines / sang, lequel est déterminé pour un patient donné par mesures simultanées des taux de glucoses dans les urines et dans le sang.

7. Dispositif selon les revendications 1 à 6, caractérisé par un calculateur pour le contrôle du doseur ou de l'indicateur.

8. Dispositif selon l'une des revendications 1 à 7 caractérisé par une entrée agissant sur le contrôle du doseur ou de l'indicateur.